# EUROPEAN PATENT APPLICATION

(11) **EP 2 859 911 A1**
(43) Date of publication of application: **15.04.2015**
(21) Application number: 13306400.6
(22) Date of filing: 11.10.2013
(51) Int. Cl.: A61M 39/02, A61B 5/1473, A61M 1/14, A61B 5/07

(54) **Vascular access port devices with incorporated sensors**

(71) Applicant: qSTAR Medical SAS, 75011 Paris (FR)
(72) Inventor: Maginess, Maxwell G., Seattle, WA Washington 98103 (US); Alvarez, Michel J., Seattle, WA Washington 98103 (US)
(74) Representative: Mackett, Margaret Dawn

(57) **Abstract**

Described herein is a vascular access port device (300) which provides both blood monitoring and providing access to a vascular element (200) implanted within a hemodialysis patient. The device (300) comprises a body (310) connectable to the vascular element (300) at one end thereof, and a cartridge (330) mounted within the body (310). The cartridge (330) comprises an electrode set (375) arranged on a distal end (340a) of a septum (340) for sensing blood chemistry, associated electronics (360, 365, 370) for processing signals sensed by the electrode set (375), and inductive antenna arrangement (380, 400) for transmitting the processed signals to a remote location.

## Description

### Field of the Invention

The present invention relates to vascular access port devices with incorporated sensors, and is more particularly concerned with vascular access port devices with the ability to sense blood chemistry and other conditions indicative of the health of the user of such devices.

### Background to the Invention

End Stage Renal Disease (ESRD) or kidney failure is commonly preceded and worsened by multiple co-morbidities as reported in U.S. Renal Data System, USRDS 2010 Annual Data Report: National Institutes of Health, National Institute of Diabetes and Digestive and Kidney Diseases, Bethesda, MD, 2010; Pritchard S.S, "Comorbidities and their impact in patients with end-stage renal disease", Kidney International, 57 Supplement 74, S100-S104, 2000; and many other available publications. Diabetes is a leading cause of such co-morbidities and coexists with ESRD in approximately 50% of dialysis patients in many countries. There is clinical controversy as discussed in Scheernthaner G, Ritz R, and Schernthaner G-H "Strict glycemic control in diabetic patients with CKD or ESRD: beneficial or deadly?" Nephrology Dialysis and Transplant, 25, 2044-2047, 2010, over the degree of desirable glycemic control for minimized overall patient risk, but in all cases optimum patient care requires systematic and frequent measurement of blood glucose levels. Another 25% of ESRD patients have co-existing hypertension and blood flow irregularities and could benefit from closer monitoring of pressure and flow physical conditions.

A wide range of patient useable devices are available to provide measurements of blood glucose and blood pressure, for example, the "One Touch Ultra" glucose meter from Johnson and Johnson, New Brunswick NJ, USA. These devices rely entirely on patient voluntary compliance and provide a blood glucose reading only for the instant of use. For the best management of ESRD patients it would be advantageous if glucose and other physiological indications could be recorded frequently and automatically with minimal intervention needed by the patient.

Treatment of patients with ESRD most often requires periodic access to the vasculature of the patient and temporarily routing a portion of the blood flow through external dialysis equipment to remove impurities normally filtered by functioning kidneys. Most commonly, dialysis is carried out on a patient up to three times a week with each treatment period lasting from between three to five hours. During these treatment sessions, blood chemistry can readily be measured, but, the sessions are too infrequent for good glucose control. Further, due to the normal blood flow being disrupted, measurement of hemodynamic properties is impractical during the treatment sessions.

The most common vascular access method for dialysis is to surgically construct a connection between an artery and a vein to form an autologous fistula (AVF). Most often, an AVF is constructed in an arm of the patient. After this connection is formed, the vein will expand in diameter under the increased blood pressure until the fistula has matured sufficiently to accommodate the blood flow rates needed for dialysis. For each treatment session, large gauge hollow needles, typically having an outer diameter of between 1.5mm to 2mm, are introduced through the skin into the fistula to remove blood from the patient for dialysis and to return blood to the patient after passage through the dialyzer equipment.

A less common variation is to place an artificial graft between an artery and vein (AVG) rather than joining them directly and then insert the dialysis needles into this graft.

Details of these access means, both AVFs and AVGs, can be found in standard texts, for example, "Hemodialysis Vascular Access and Peritoneal Access" by C Ronco and NW Levin, S Karger AG, Basel 2004.

For a patient, the repeated use of needles is a significant issue for pain as well for many side effects, including infections, needle displacements, vessel damage and hematomas. Most often, treatment requires attendance at a clinic with careful supervision during the dialysis sessions.

Vascular access port devices may be used to overcome the issues associated with the use of dialysis needles. Several vascular access port devices have been disclosed in which the port is implanted through the skin of the patient to form a permanent connection into an artificial or natural blood vessel. Examples of such devices are described in US-A-4349021, US-A-4421507, US-A-4534761, US-A-4654033, US-A-4496350, US-B-6231541 and US-B-7223257. These devices provide means for access to the vascular system of the patient, as needed, without the need for repetitive puncturing of the skin and/or blood vessel or graft.

In relation to the continuous measurement of blood glucose levels, an extensive overview of methods and devices for such measurements can be found in *"*In Vivo Glucose Sensing" by D.D. Cunningham and J.A. Stenken, Wiley, New Jersey, 2010. For best accuracy and fast response to changes, glucose level measurements are most desirably made directly on whole blood. However, present methods to achieve this require skin and blood vessel breaches for the insertion of catheters into blood vessels, for example, as discussed by Skjaervold N K, et al., "Continuous Measurement of Blood Glucose" Anesthesiology 114, pages 120 to 125, 2011, and, in US-A-5704554. The use of needles is also described in US-A-2009/0264719. More often, for continuous measurement, blood glucose levels are inferred from interstitial fluid levels sampled either percutaneously, for example, as described in US-A-6360888, or with subcutaneously implanted sensors such as disclosed in EP-B-1214586.

It should be understood that, in the present context, the term "continuously" means that measurements are taken at sufficiently frequent intervals to accurately track temporal changes in blood glucose or other analytes. This usually requires sampling at least several times per hour as described by Gough D A, Kreutz-Delgardo K, and Bremer T in "Frequency characterization of blood glucose dynamics", Annals of Biomedical Engineering, 31, pages 91 to 97, 2003.

The most common method of blood glucose sensing uses an enzyme-based electrochemical reaction with amperometric readout as discussed by Heller A, "Implanted Electrochemical Glucose Sensors for the Management of Diabetes", Annual Review of Biomedical Engineering, pages 153 to175, 1999. Similar means may be used for sensing other blood chemistry including urea and creatinine as described by Ho W O et al., "Electrochemical Sensor for Measurement of Urea and Creatinine in Serum Based on as Impedance Measurement of Enzyme-Catalysed Polymer Transformation" Annals of Chemistry, 71, pages 1940 to 1946, 1999. With extended use, these sensors degrade in sensitivity and stability.

Published research indicates a variety of investigations and design modifications to such sensors in order to achieve improved performance and longevity. Continuous operation up to nearly a year is reported in limited cases, for example, by Renard E, "Implantable Continuous Glucose Sensors", Current Diabetes Review, 4, pages 169 to 174, 2008. However, practical on market devices such as the Dexcom Seven™ Plus, (Dexcom Inc. San Diego CA, USA) using percutaneous sensors, currently require device replacement every 5 to 7 days, imposing a high burden of patient self-maintenance and cost.

Catheter mounted or implanted sensors using any technique present additional problems for ESRD patients since access or replacement requires additional surgery with attendant procedural and infection risks.

Percutaneous and subcutaneous sensors are subject to the natural reaction of the human body to foreign bodies and become walled off by collagen capsules, greatly affecting sensing performance. Sensors maintained in contact with flowing blood typically function for longer periods, allowing extended use. For example, E Renard in "Implanatable continuous glucose sensors", Current Diabetes Review, 4, pages 169 to 174, 2008, reports more than double the operational life of intravenous glucose sensors compared to subcutaneous ones.

US-B-7070591 discloses a device for monitoring physiological conditions while providing a limited vascular access. The vascular access port is implanted completely subcutaneously and is therefore not adapted for use in hemodialysis. Further, while the monitoring device is mechanically connected to the port to allow implanting in the same surgical procedure, there is no functional integration or communication between the port and the monitoring device.

US-B-6442413 (together with US-B-6895265 and US-B-7033322) provides for the use of an electrochemical glucose sensor maintained in blood contact where the sensor is affixed to an implanted vascular stent or graft-stent. No provision is made for repeated vascular access such as required for hemodialysis, for replacement of the sensing chemistry or against any device failures. Surgery is required specific to the device implantation.

US-B-7762977 describes a vascular access device with provision for blood flow measurement. This flow sensing device uses a catheter portion of the overall device, but no port is included.

US-A-2005/0131305 discloses means for monitoring the temperature of circulating blood by use of a thermally sensitive liquid crystal. The liquid crystal is intended to form an external cover for a blood access device such as that described in US-A-6436089. Other blood parameter or chemistry measurements are possible, but each measurement requires a particular cover composition.

US-A-5120313 discloses a device for blood pressure measurement via a percutaneous access port. No measurements are provided for other physiological conditions and there are no means for use of the same assembly for hemodialysis.

US-B-8352011 achieves blood condition monitoring by sensors inserted in external blood loops, such as those formed during an actual dialysis procedure, but offers no means for measurement of patient condition when not undergoing treatment.

None of the devices described above form a combined hemodialysis/sensor device.

### Summary of the Invention

In light of the drawbacks and limitations described above in relation to current devices for the management of diabetic and other comorbidities commonly present in ESRD patients, it is an object of the present invention to provide a blood monitoring system which allows systematic recording of critical patient physiological measurements using a permanently implanted dialysis vascular access port device.

It is another object of the present invention to provide a blood monitoring system in which data relating to blood chemistry, for example, glucose levels and hemodynamic factors, can be determined and transmitted to a remote monitoring station, such as, a doctor's office, clinic or hospital.

In accordance with a first aspect of the present invention, there is provided a cartridge for use in a vascular access port device operatively connected to a vascular component of a patient, the cartridge having a distal end and a proximal end, the cartridge comprising:-
an internal cavity adapted to receive a cannula for connection to an external dialysis machine; and
a re-sealable element located at the distal end of the cartridge and operable for providing cannula access to the vascular component when located in the vascular access port device, the re-sealable element having an external surface in contact with blood in the vascular component;
**characterized in that** the cartridge further comprises a sensor arrangement located on the external surface of the re-sealable element, the sensor arrangement being adapted for generating electrical signals indicative of measurements taken thereby; an integrated circuit for processing the generated electrical signals; and an inductive coil element for transmitting the processed electrical signals to an external location.

By providing such a cartridge, it is possible to monitor blood chemistry and/or other conditions on a regular basis and transfer data relating to these measurements to a remote location for analysis and/or monitoring.

The term "vascular component" as used herein refers to any natural or artificial vessel forming part of the blood circulatory system of a patient and through which blood flows. The vascular component may be a vascular graft, such as, for example, an arterio-venous graft or an autologous fistula.

The term "vascular access port device" as used herein refers to a device which can be percutaneously positioned within a patient and which is connected to a vascular component to provide periodic access to blood flow within the component for hemodialysis, for example. Such a device comprises a body portion having a distal end, defining a port and which is connected to the vascular component, and a proximal end on the surface of the skin through which access is provided for cannulae, needles and the like, without further need to penetrate the skin, surrounding tissue or another vascular component. The port is closed by a cartridge or other suitable means to prevent blood loss when the device is not use.

The cartridge advantageously further comprises a flexible circuit for providing connections between the sensor arrangement, the integrated circuit, and the inductive coil element. A power supply may also be connected to the integrated circuit by way of the flexible circuit. In one embodiment, the power supply comprises a battery.

The inductive coil element may be located in an upper portion of the cartridge cavity around an inner wall thereof, In one embodiment, a liner, preferably made from a high magnetic permeability ferrite material to increase coil inductance and coupling to external readout devices, is located within the upper portion of the cavity and inside the inductive coil element. If a ferrite material is used for the liner, it may be necessary for the internal surface thereof to be coated to prevent damage to the ferrite material due to repeated insertion and removal of a dialysis cannula, for example.

A substantially rigid insert may be located within a lower portion of the cavity on the inner wall thereof which is operable for guiding the insertion and removal of a cannula into and out of the cartridge.

In one embodiment, the sensor arrangement comprises an electrode set with a sensing electrode, a counter electrode and a reference electrode. Each electrode preferably has a coating appropriate to its function. In the case where the cartridge is to be used for blood glucose sensing or monitoring, the sensing electrode is coated with an enzyme which is sensitive to the presence of glucose.

The re-sealable element, for example, a blood contacting plug or septum, may be provided with self sealing slits or other like provision at its distal end for leak free penetration by one or more cannulae adapted for substantially diverting blood circulation from the connected vascular component through an external dialysis machine during a treatment session. A central, open passage (or internal cavity) is provided which allows the insertion of such cannulae from the proximal end of the cartridge to the distal end thereof.

A further electrical contact may be provided on the cartridge for forming a common connection with the vascular access port device and for shielding the sensor arrangement against interfering signals. In this case, the shielding is provided by a body portion of the vascular access port device when the cartridge is mounted therein.

In accordance with another aspect of the present invention, there is provided a vascular access port system comprising:-
an implantable body portion having a distal end and a proximal end and being operable for percutaneous implantation with the distal end defining an opening into a vascular component;
a retention portion formed on an outer wall of the implantable body which is adapted for anchoring the implantable body in place relative to the vascular component; and
a cartridge as described above located within the implantable body portion, the sensor arrangement being located on an external surface of the re-sealable element being positioned at the distal end of the implantable body portion for contact with blood flow through the vascular component.

A mechanically compliant layer is preferably provided on outer wall of the implantable body. This has the advantage that the tissue surrounding the implantable body is protected from any shock created by the insertion of a cannula into the body and its subsequent removal therefrom.

Additionally, an infection resistant tissue interface layer is formed over the mechanically compliant layer on the outer wall of the implantable body.

A vascular access port system in accordance with the present invention provides positioning of a monitoring device substantially within the implantable body with an active sensor arrangement on the distal end providing a highly effective means for maintaining continuous contact with the blood flow through the vascular component to which the device is attached or connected.

By providing an active sensor arrangement within the cartridge on the distal end of the re-sealable element or septum, when the re-sealable element needs to be replaced to maintain dialysis access without blood leakage, the sensor arrangement can also be replaced. This has the advantage that sensor fouling, plaque accumulation, and depletion of sensing chemicals can readily be corrected. In the case of failure of a device, the cartridge can readily be replaced without having to disturb the implanted body and/or its connection to the vascular component. In addition, by having a modular system of this type, the sensor capabilities can readily be updated by simply exchanging the cartridge within the port device at a suitable time, for example, at the next replacement interval.

The present invention thus provides a device for long-term, systematic measurement of patient physiological status including blood analytes, particularly as applied to ESRD patients undergoing hemodialysis. Extracorporeal blood circulation during dialysis allows for independent calibration of the blood chemistry sensors by external instrumentation. Moreover, the system of the present invention does not rely on the patient monitoring their own blood chemistry and no additional devices or equipment are required to obtain a blood chemistry measurement.

A preferred embodiment of the system comprises a percutaneously implanted vascular access port adapted to use for hemodialysis treatment via a tubular central lumen having a distal end in communication with the blood flow in a vascular component, the port comprising an interior structural body formed from titanium or other durable, biocompatible material capable of being fabricated and retaining precise lumen dimensions over extended use cycles; an external covering preferably of silicone, of thickness and shape as to provide mechanical retention and a compliant buffer between the port body and surrounding tissue; and a further infection resistant external covering such as disclosed in US-B-8372432.

As the cartridge is readily replaceable, a passive cartridge may be used during periods when blood chemistry measurements are not needed. A passive cartridge still provides access to the vascular component for hemodialysis and also provides secure sealing of the port against blood leakage from the attached vascular component. The cartridge, whether it be a sensing cartridge in accordance with the present invention or a passive cartridge, can readily be removed and replaced as required with a fresh cartridge by means of a keyed tool which prevents accidental removal of the cartridge from the port body and consequent unnecessary loss of blood.

During exchange of cartridges, temporary provision is made to block blood flow within the vascular component. As an example, a balloon-tipped catheter introduced into the vascular component via the septum slit provided for cannula insertion. Once the balloon is placed and inflated the cartridge may be removed by sliding over the catheter shaft and a replacement introduced over the catheter shaft, and once in position, the balloon is deflated and removed from the device and the septum re-seals after its removal.

To maintain a competent seal against blood leakage the cartridge will typically need to be exchanged at intervals of 40 to 80 dialysis sessions or every 3 to 6 months. Thus it is desirable that the monitoring sensors operate for a like period.

The vascular port access system further comprises a cap locatable on the implantable body portion, the cap having a rim portion operable for engaging with a groove portion formed at the proximal end of the implantable body portion. The cap preferably includes an inductive antenna coil, the inductive antenna coil being alignable with the inductive antenna coil in the cartridge when the cap is attached to the implantable body portion. The cap may further comprise a high magnetic permeability liner associated with the inductive antenna coil.

In accordance with a further aspect of the present invention, there is provided a blood monitoring system for monitoring blood parameters of a hemodialysis patient, the system comprising:-
a vascular port access system as described above implanted in the hemodialysis patient and operable for providing signals indicative of blood parameters of the patient; and
a processor operable for receiving the signals from the vascular port access system, for processing the received signals to provide data relating to the blood parameters, and for transmitting the data to a remote monitoring station.

In one embodiment, the remote monitoring station may be a data receiving and readout device which is preferably located externally on the patient, the device being adapted to receive transmitted data wirelessly from the cartridge located within the vascular port access system. Functions of this external device may also comprise without limitation, transmission of data to a further device, programming of the cartridge function, or wireless transmission of energy to maintain a battery in the cartridge.

In another embodiment, the remote monitoring station may be a hospital or an office of a suitably qualified medical practitioner to which data may directly be transmitted.

The processor may comprise an interface assembly, which, in one embodiment, may be wirelessly connected to the vascular access port system. In an alternative embodiment, the processor may comprise a transceiver device connected to the interface assembly by a communications link. The transceiver device may comprise, in one embodiment, a smart phone. In this case, the smart phone may run an appropriate app to provide the data transfer between the cartridge in the vascular access port system and the external location.

### Brief Description of the Drawings

For a better understanding of the present invention, reference will now be made, by way of example only, to the accompanying drawings in which:-
Figure 1 illustrates a conventional prior art vascular access port device connected to the vascular system of a patient;
Figure 2 illustrates an embodiment of a vascular access port system in accordance with the present invention;
Figure 3 is a cross-sectional detailed view of a sensing cartridge shown schematically in Figure 2;
Figure 4 is a cross-sectional view of a vascular access port device in accordance with an embodiment of the present invention taken along lines B-B of Figure 3;
Figure 5 illustrates an exemplary arrangement of sensor electrodes within the vascular access port device shown in Figure 3;
Figure 6 illustrates the connection of the vascular access port device in accordance with the present invention to an arterio-venous graft (AVG) or an autologous fistula (AVF);
Figure 7 illustrates a vascular access port device of Figures 2 to 6 forming part of a monitoring system; and
Figure 8 illustrates a block diagram of a processing and communications system for the vascular access port device illustrated in Figures 2 to 7.

### Description of the Invention

The present invention will be described with respect to particular embodiments and with reference to certain drawings but the invention is not limited thereto. The drawings described are only schematic and are non-limiting. In the drawings, the size of some of the elements may be exaggerated and not drawn on scale for illustrative purposes.

It will be understood that the terms "vertical" and "horizontal", "upper" and "lower" and "proximal" and "distal" as used herein refer to particular orientations of the Figures and these terms are not limitations to the specific embodiments described herein. In particular, the term "distal end" is used to refer to a portion of a device or other element that is located closer to the connection with the vascular component than another portion, the "proximal end", of the same device or other element, generally located closer to the patient skin line.

Device components which are the same bear the same reference numerals throughout the following description.

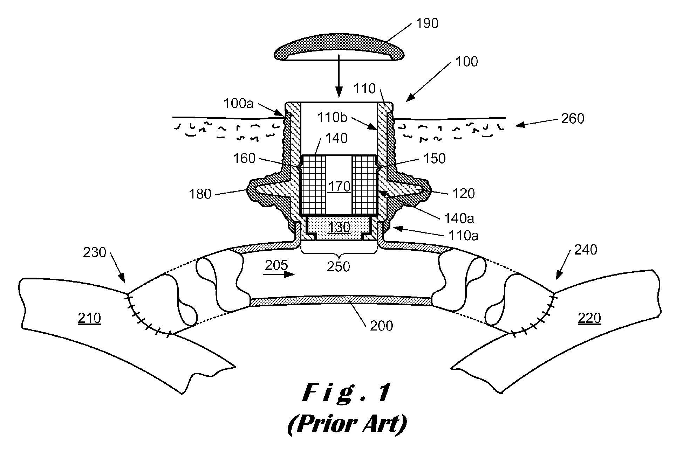

Referring initially to Figure 1, a generalized cross-sectioned representation of a conventional (or prior art) vascular access port device 100 is shown mounted on an artificial graft 200 between an artery 210 and a vein 220. Blood flow then occurs in the direction indicated by arrow 205. Such an artificial graft 200 is termed an "AVG" hereinafter. The AVG 200 has a first end 230 connectable to the artery 210 and a second end 240 connectable to the vein 220 when implanted into a patient represented by skin 260, artery 210 and vein 220. The AVG 200 has an opening 250 on which is mounted the port device 100. The ends 230, 240 of the AVG 200 are connected respectively, by anastomosis, in a conventional surgical manner to the natural artery 210 and vein 220 of the patient.

The device 100 comprises a body 110 connectable distally into the opening 250 of the AVG 200, the body 110 typically having an annular flange portion or portions 120 formed on its outer wall for anchoring the device 100 in place once surgically implanted percutaneously into the patient. As shown, a proximal or upper portion 100a of the device 100 protrudes through the skin 260.

A septum 130 is provided within a distal or lower portion 110a of the body 110 and adjacent to the distal port opening 250 as shown. The septum 130 provides a seal against blood leakage through the opening 250. Septum 130 may comprise self-sealing slits which allow penetration by one or more cannulae into the AVG 200 for blood access and connection to a dialysis machine. It is well known to design such septa to maintain seal integrity against repeated insertion and withdrawal of a cannula during multiple treatment sessions. However, periodic replacement of the septum 130 is necessary. Exemplary prior art replacement means are disclosed in US-B-7223257, US-A-4405320 and US-A-4349021.

An insert member 140 is located within the body 110 above the septum 130. In a typical arrangement, the insert member 140 may be retained in a position relative to the body 110 by means of an annular projection 150 formed on an outer wall 140a thereof which engages with an annular recess 160 formed in an inner wall 110b of the body 110. The insert member 140 has a substantially central cylindrical hole or lumen 170 extending its entire length which is operable for receiving tubing or a cannula (not shown) during a dialysis session. Further projection of the tubing or cannulae through the septum 130 to provide dedicated channels through which blood is removed from and returned to the patient.

Outer wall of the body 110 is often covered with a tissue interfacing material 180 for integration with the subcutaneous tissue (not shown) of the patient. Prior art materials which can be used as the tissue interfacing material include porous titanium (as disclosed in US-A-4405319), pyrolitic carbon (as disclosed in US-A-4534761) and Dacron velour (as disclosed in US-B-6524273). [Dacron is a registered trademark of INVISTA.] More recently, bio-integrating scaffold materials, such as, those disclosed in US-B-8318193, EP-B-1670385 and US-B-8372423, have become available to provide the tissue interfacing material. In addition, such materials may also provide improved infection control such as described in US-A-2012/0053673 and US-A-2013/0236502.

A cover 190 is provided for sealing the body 110 when the device 100 is not being used, the cover 190 typically providing a continuous surface with the skin 260 in between dialysis sessions.

In use, the device 100 becomes embedded into the skin 260 and subcutaneous tissue (not shown), with the flange portion 120 providing a mechanical attachment within the subcutaneous tissue. The flange portion 120 may be further adapted to retain sutures placed into the adjacent subcutaneous tissue.

In some cases, a vascular access port device may be connected into an autologous fistula (commonly referred to as "AVF"), for example, in a manner similar to that disclosed in US-A-4421507 or via a vessel patch sealed to the distal end of the vascular access port.

It will be understood that, whilst Figure 1 is representative of prior art or conventional hemodialysis vascular access port devices, many variations have been disclosed to achieve similar overall functionality. These include, as examples, use of valves for access and control of blood flow, insertion of catheters via the port, alternative means to secure the blood seal, AVG connection to a distal projection of the port body in the form of an inverted "T" fitting and many differing port body shapes and cover designs.

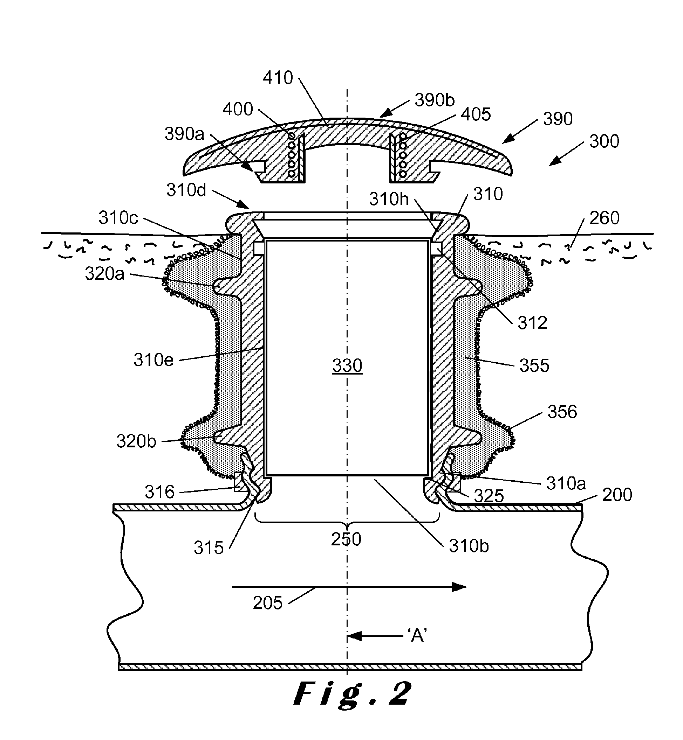

Turning now to Figures 2 to 5, a vascular access port device 300 in accordance with the present invention is shown. The device 300 comprises a generally tubular body 310 (similar to body 110 shown in Figure 1) which is connectable to an opening 250 into an AVG 200 at a distal end 310a thereof. As described above, the vascular component to which the vascular access port device 300 is connected may be an autologous fistula or AVF (not shown) instead of an AVG.

Distal end 310a of the body 310 defines an opening 310b through which connection to the opening 250 in the vascular component 200 can be made. The distal end 310a is shaped to have a groove arrangement 315 adapted to provide a seal between the AVG 200 and the body 310 and which is secured by means of an annular clamp 316 engaging the walls of opening 250 with a complementary groove arrangement (not shown in detail).

Two annular flange portions or protrusions 320a, 320b are formed on outer wall 310c of the body 310 for anchoring the device 300 in place once implanted within the patient under his/her skin 260. As shown, a proximal end or upper portion 310d of the body 310 protrudes through the skin 260. Although two annular flange portions or protrusions are shown in this specific embodiment, it will readily be appreciated that one or more annular flange portions or protrusions may be implemented depending on the specific configuration of the body 310.

The generally tubular body 310 may be made from titanium for biocompatibility, durability and consistent dimensions to ensure sealing against blood leakage and to maintain a well-controlled fit for a dialysis cannula. In one embodiment, the body 310 may have a substantially circular cross-section, and in another embodiment, the cross-section may be substantially elliptical. In each case, the body 310 is symmetrical about a plane extending through an axis indicated as 'A' in Figure 2. However, it will be appreciated that other cross-sections are possible, for example, any suitable symmetrical or asymmetrical shape is possible.

It may also be envisaged that the external cross-section of the body 310 may be chosen for its ability to anchor itself within the percutaneous tissue of a patient whilst the internal cross-section may be different to ensure a snug fit between the inner wall 310e of the body and a cartridge (as will be described in more detail below) inserted into the body 310 to prevent blood loss when the system is implanted within a patient.

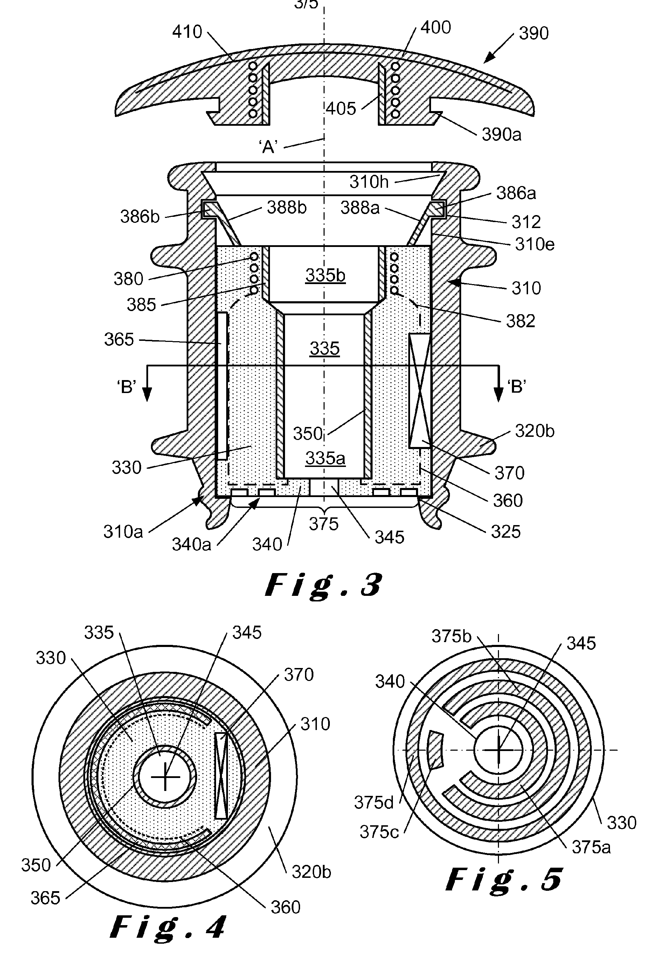

A cartridge 330 is shown located within the body 310 in Figure 2 but will be described in more detail with reference to Figures 3 to 5 below.

Returning to Figure 2, outer wall 310c of the body 310 (together with the annular flange portions or protrusions 320a, 320b) is covered with a mechanically compliant layer 355 having an outer covering 356. The mechanically compliant material 355 and the outer covering 356 comprise bio-interface materials for biocompatibility with the subcutaneous tissue (not shown) of the patient. Such a compliant layer 355 reduces the transfer of mechanical stresses generated during cannula insertion and other dialysis treatment activities. The outer covering 356 may preferably be of an infection resistant type as disclosed in US-B-8372423 and US-A-2013/0236502. Other suitable constructions for the tissue interfacing materials 355, 356 include those described above with reference to Figure 1.

An annular groove or cut-out 312 is provided in the inner wall 310e of the body 310 which is used for retention of the cartridge 330 when it is located within the body 310 as will be described in more detail below with reference to Figure 3.

In Figure 3, the cartridge 330 is shown extending generally from the distal end 310a and opening 310b towards the proximal end or upper portion 310d of the body 310. As described above, the cartridge 330 is sized to fit snugly with inner wall 310e of the body 310 and includes a cavity 335 closed at its distal end by a septum 340. The periphery of the septum 340 is located against a shoulder 325 formed in the distal end 310a of the body 310 and provides a seal to prevent blood flow from the distal port opening and into the interior of the device body 310.

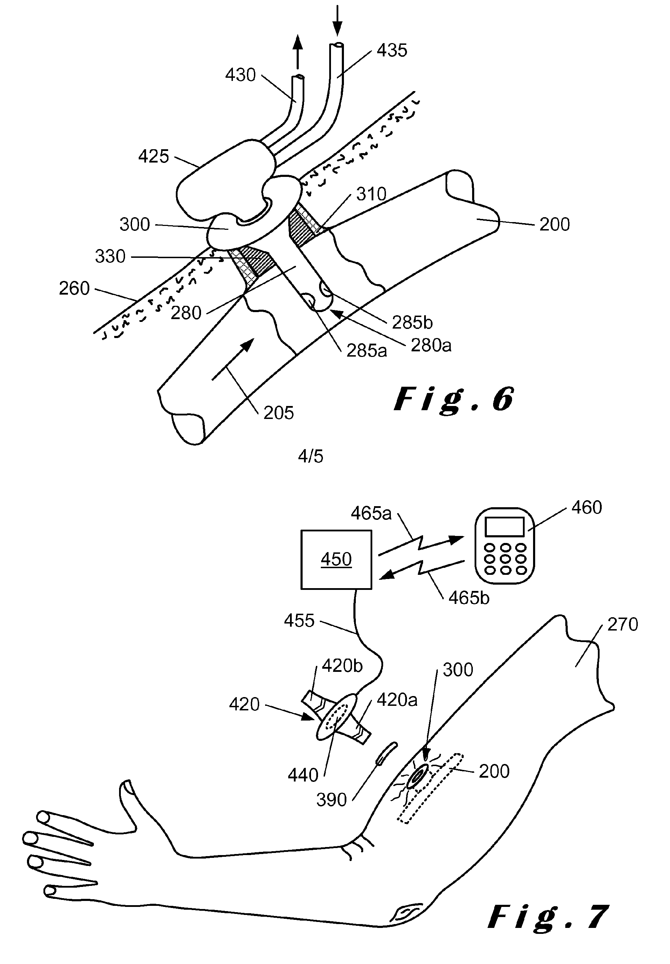

The cavity 335 comprises a lower portion 335a and an upper portion 335b. In the lower portion 335a, the cavity 335 is adapted to receive and guide a cannula 280, as shown in Figure 6, to penetrate septum 340 through formed slits 345 for access to blood flowing in AVG 200 during hemodialysis. On withdrawal of the cannula 280, slits 345 reseal to prevent blood leakage from AVG 200.

As shown in Figure 3, the cartridge 330 is symmetrical about axis 'A', that is the axis of the body 310. The cartridge 330 is provided with upper portions 386a, 386b which are connected to the main cartridge by means of respective arms 388a, 388b. The upper portions 386a, 386b engage with the annular groove or cut-out 312 is provided in the inner wall 310e of the body 310.

Upper portions 386a, 386b and their associated arms 388a, 388b may comprise two separated diametrically opposed portions which extend from the top of the cartridge body. However, it will be appreciated that there may be any suitable number of upper portions and associated arms arranged in any suitable configuration.

Cartridge 330 including septum 340 may be made from a suitable long term implant silicone material such as Nusil MED 4735 (Nusil Technologies LLC, Carpentaria California, USA) suited to both the sealing function and to protective encapsulation of sensor components. For retention of cartridge shape and accurate, low friction cannula guidance, cavity 335 may be supported by a generally tubular insert 350 formed from a stiff or rigid plastic material, such as polyether ether ketone (PEEK), bonded to the inner wall of cavity 335 in the lower portion 335a thereof. As shown in Figure 3, the upper portion 335b of the cavity 335 may have a larger diameter than the lower portion 335a, the lower portion 335a guiding the cannula 280 during insertion and removal as described above.

In addition, the arms 388a, 388b may also serve to guide the insertion of the cannula 280 whilst defining a tapering region which allows for the accommodation of flaring of the cannula internal lumens (not shown) into hub 425 (Figure 6) with a minimal projection above the skin line 260.

Shoulder 325 of body 310 is sized to retain the cartridge 330 and to leave sufficient access for a sensor arrangement or sensor set 375 to blood flow within the vascular component as will be described in more detail below. For controlling cannula insertion for dialysis through the cartridge 330 and septum 340 into AVG 200, slits 345 provide a controlled insertion force while retaining a seal against blood loss. Minimum thickness of the septum 340 is determined by openings 285a, 285b in the cannula 280 (Figure 5) for collection and return of blood after dialysis. A representative septum thickness dimension is between 3mm and 4mm. Such self sealing means are well known and variations in material, slit geometry and other details may be made without limitation of function.

In accordance with the present invention, the cartridge 330 includes a flexible circuit 360, a flexible battery 365 and an integrated circuit 370. For greater clarity the arrangement of components are shown in Figure 4 which is a section view taken along lines B-B of Figure 3. In Figure 4, the battery 365 is mounted within the cartridge 330 and is connected to the integrated circuit 370 by means of the flexible circuit 360. The flexible circuit 360 is also connected to the induction coil 380 as shown in Figure 3 at 382.

A sensor arrangement or set 375 is provided at distal end 340a of the septum 340 and is located around the slit 345 as shown in Figure 5. In one embodiment, the sensor set 375 comprises a blood glucose sensing electrode 375a, a counter electrode 375b and a reference electrode 375c. The operation of the flexible circuit 360, battery 365, integrated circuit 370 and the electrodes 375a, 375b, 375c will be described in more detail below. In addition to the electrodes 375a, 375b, 375c, a surface electrode 375d may also be provided on the septum 340 and is located on a peripheral area thereof in pressure contact with shoulder 325 of body 310. The purpose of the surface electrode 375d is described in more detail below.

In addition, an inductive antenna or solenoid coil 380 is provided within the cartridge portion 330e surrounding the upper portion 335b of the cavity 335. A cylindrical core 385 of ferrite material is further provided inside the coil 380 to control its inductive properties and provide increased magnetic coupling to the corresponding antenna components 400, 405 and 410 in cap 390 or component 440 in cover 420 (Figure 7). For protection and biocompatibility, both the inductive antenna coil 380 and the liner 385 of ferrite core material are enclosed within the material of cartridge 330. (Ferrite materials are readily procured from many suppliers, for example Ferroxcube International Holdings B.V. Eindhoven NL.).

A cap 390 is provided for sealing the body 310 when the device 300 is not being used for dialysis. The cap 390 includes a tab or rim portion 390a which engages with a groove 310h formed in the proximal end 310d of the body 310 as shown. Insertion and removal of the cap 390 is achieved by flexing the cap to engage and disengage the tab/rim portion 390a with and from the groove 310h.

In one embodiment, the cap 390 is formed entirely from a suitable polymer material with no effect on the transmission performance. This provides for an inexpensive item that may be removed and replaced at any time for patient port site care. It may be the case that the cap 390 needs to be replaced after each hemodialysis session to ensure adequate sealing with the body 310 to prevent the ingress of foreign material.

In another embodiment, cap 390 may also include a ferrite core 405 arranged for magnetic coupling to the field produced by coil 380 and core 385. For best coupling, the adjacent ends of the ferrite cores 405 and 385 should be axially aligned and in close proximity when cap 390 is in place over the body 310 of device 300.

In a further embodiment, a further coil 400 is provided in the cap 390 surrounding core 405 and may be further arranged to excite antenna elements 410 in the cap 390.

Coupling may be yet further improved by providing for portions of core 405 and coil 400 to be formed with a smaller diameter so as to be adapted to be inserted into upper portion 335b to lie co-axially with core 385 and coil 380.

These arrangements functionally extend the effective size of coil 380 outside the shielding effect of the port body 310, thus increasing external radiation. In this way, the data transmission range may be increased for a given power from integrated circuit 370 and battery 365 providing more flexibility in locating a monitoring assembly as will be described in more detail below.

Antenna efficiency within the vascular access port device 300 may be further improved by tuning one or both inductive antenna coils 380, 400 (or coil 400 and antenna elements 410) to form a resonant coupling in a manner similar to a Tesla coil. In the example of blood glucose measurement, data readout rates are low, typically about 16 bits of data per sample, with each sample being taken at 10-minute intervals or longer. At such data readout rates, the data may adequately be transmitted within a reduced bandwidth provided by a resonant coil configuration.

Detailed design information for such wirelessly coupled devices is readily available in the field of radio frequency identification (RFID) tag reading, and is described, for example, in *"*Antenna Circuit Design for RFID Applications" by Y. Lee, Application Note S00710C, Microchip Technology Inc. 2003.

It will be appreciated that many different materials may be used for the body 310 of the device 300 with other components being made with particular details of shapes and means to realize the essential requirements of sealing, retention of the cartridge, placement and retention of the cap, connection to the AVG etc., without compromising the use of the vascular port access device for hemodialysis procedures.

As described above, the cartridge 330 comprises a self-contained blood chemistry or physical condition sensors system, exemplified in the form of the sensor set 375, with wireless communication capability to transmit the data obtained from the sensors. As described with reference to Figures 2 to 5, the cartridge 330 has the ability to measure blood glucose, but it will be appreciated that the present invention is not limited to the measurement of blood glucose levels, and other blood parameters may be measured with a cartridge having appropriate sensors, whilst providing a vascular access port for hemodialysis.

In one embodiment of the cartridge 330, electrodes 375a, 375b and 375c are provided integral to the distal end 340a of the septum 340 (as shown in particular in Figure 5) surrounding the slit 345 in the penetrable septum 340 and are disposed for continuous contact with blood flow within the AVG 200. For amperometric detection of the blood glucose levels using a sensing electrode 375a, it is well known to provide a coating thereon comprising at least a glucose reactive enzyme layer, typically, of glucose oxidase, and a mediator layer to control the reaction rates. Counter electrode 375b comprises a plain platinum surface, and reference electrode 375c comprises a silver/silver chloride surface.

The arrangement, composition and electrochemical response of sensing electrodes as described herein is originally disclosed in US-A-3539455 and US-A-4721677 and is also described in numerous improvements of which US-A-5330643, US-B-7336984 and US-B-7722913 are representative. Variations in electrode geometry and coatings may be made without alteration to the required functionality. Alternative sensing means such as described by Piechotta G, Albers J and Hintsche R, "Novel Micromachined Silicon Sensor for Continuous Glucose Monitoring", Biosensors and Bioelectronics, 21, pages 802 to 808, 2005, or optical based sensing means as disclosed in US-B-6330464 may be substituted.

The sensor set 375 is operatively connected to integrated circuit 370 via flexible circuit 360. All connections and circuits are contained within impermeable protective material of cartridge 330. The same silicone material, as described above, which is appropriate for forming the septum 340 of the cartridge 330 is often suitable for housing the connections and circuits and provides a simple unitary construction for the cartridge 330.

One arrangement of the integrated circuit 370, the flexible circuit 360 and flexible battery 365 within the cartridge 330 within the body 310 is shown in cross-section by Figure 4, the electronic components being powered by the flexible battery 365. It will readily be appreciated that other arrangements are possible.

Battery 365 is preferably of a high energy density type such as described in Bates, J. B. et al., "Thin Film Rechargeable Lithium Batteries for Implantable Devices", ASAIO J. 43, 644-67, 1997. Such flexible batteries are in common use for "smart" credit cards and the like. By setting the implant for less frequent sensing while still meeting the desired sampling requirements as referenced above, sufficient energy for use during the entire period between required cartridge replacements may be obtained from a non-rechargeable battery.

The integrated circuit 370 comprises analogue circuitry for interfacing with the electrode set 375 using well known electronic designs such as those described, for example, by Yamazaki, T et. al. "Smart Integrated Sensor for Multiple Detection of Glucose and L-Lactate Using On-Chip Electrochemical System". Journal of Sensors, 2011, ID 190284.

Sensor operation may be protected from external electronic interference by providing an ohmic connection between the common or ground of this interface or analogue circuitry and the body 310 of the device 300. Suitable ohmic connection means may be provided by means of the surface electrode 375d provided on the septum 340 which is connected to the integrated circuit 370 via a trace on flexible circuit 360.

The integrated circuit 370 also provides analog-to-digital conversion of the resultant glucose level measurement signal; short term memory for recent measurements; control logic, including timing means for analyte sampling and transmission intervals; and a transmit/receive circuit for communicating the measurements to external devices. In certain embodiments, the integrated circuit 370 may also receive power and control signals from external devices.

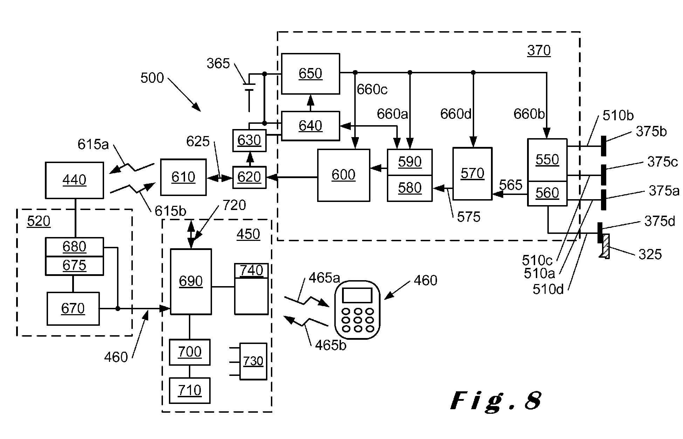

Certain electronic capabilities required within cartridge 330 may preferably be implemented separately from integrated circuit 370. An exemplary partitioning of the total functionality is described below with reference to Figure 8.

As shown in the embodiment of the vascular access port device 300 in accordance with the present invention illustrated in Figure 3, integrated circuit 370 is further connected to inductive antenna coil 380 located within cartridge 330. Such an inductive arrangement may, for example, be realized by a solenoidal formation of conductors on the flexible circuit 360. It will be appreciated that, although the antenna 380 is represented as an inductive antenna coil in Figure 3, it could be provided in any other suitable form. In order to minimize alteration of inductive properties from the metallic components of the device 300, it is preferable that the inductive antenna coil 380 be disposed relatively centrally with respect to the body 310 as indicated in Figure 3. Guidance on the design of such coils located within electrically conductive shields may be found in "Radiotron Designers Handbook" by F. Langford-Smith, Iliffe, 4th Edition, 1957.

In Figure 6, the device 300 in accordance with the present invention is shown connected to an AVG 200 with a cannula 280 in place. The cap 390 is removed from the body 310 to provide access to the cavity 335 within the cartridge 330. The cannula 280 is inserted into cavity 335, through the septum 340 and into the AVG 200 as shown. Openings 285a, 285b formed in distal end 280a of the cannula 280 allow the flow of blood into and out of the cannula 280. As shown, blood flow within the AVG 200 is in the direction indicated by arrow 205. The cannula 280 is connected to a hub 425 having dialysis tubes 430, 435 connected thereto, tube 430 removing blood from a patient via the AVG 200 and tube 435 returning treated blood to the patient via the AVG 200. The dialysis tubes 430, 435 are connected to appropriate portions of a dialysis machine (not shown). It will be appreciated that the cannula 280 and its associated openings 285a, 285b are suitably connected to the dialysis tubes 430, 435 by means of internal lumens (not shown). It will also be appreciated that each dialysis tube 430, 435 is connected to only one of the openings 285a, 285b so that there is no cross-flow within the device between blood being extracted and treated blood being returned.

Preferably, a notch (not shown in Figure 6) is provided in body 310 with a corresponding protrusion from cannula 280 to ensure a correct orientation between openings 285a, 285b in the cannula and the blood flow direction as indicated by arrow 205.

The proximal end 310d of body 310 of device 300 may typically project above the patient skin line by between 1mm and 5mm, and is covered when the port is not being used for dialysis, or undergoing cartridge replacement, by cap 390 adapted to prevent entry of any foreign material.

For use in established hemodialysis procedures, the port device and connected vascular component both need to provide certain functional and dimensional capacities. To ensure the blood flow rates needed for efficient hemodialysis, the vascular component will often have an internal lumen between 4mm and 8mm in diameter. In a patient without other circulatory compromises, such a vascular component will provide for a flow rate of between about 600ml/min and about 800ml/min. In dialysis use, between 200ml/min and 400ml/min of this flow will typically be diverted through the external dialyzer. To sustain this flow, a suitable cannula for insertion into the blood conduit has a lumen with a cross-sectional area of between about 1.5mm² and about 2 mm² for each flow direction. Two such lumens (for example, as back to back "D" sections) can be fitted within a single device of about 3mm to 4mm outer diameter.

To provide adequate sealing when penetrated by a cannula of about this size, the face of septum 340 exposed to blood flow needs to be between about 6mm to 10mm in diameter. This further requires that, where the vascular component comprises an AVG connected to the port, the opening needs to be expanded to a similar diameter to allow secure attachment to body 310. US-A-4512761 discloses exemplary means to realize this. Where the vascular component comprises an AVF, a similar expansion may be realized by an inserted vascular patch of designed with a standard material such at that described at: http://atriummed.com/EN/Vascular/VascularPatches.asp.

The device body 310 has a length from skin line to vascular component connection typically of about between 12mm and 15mm. These dimensions provide guidance for the geometric areas and volumes available for the implementation of a blood glucose sensor or other sensor capabilities within the compass of a functional dialysis access port.

Figure 7 illustrates the device 300 in place in an arm 270 of a patient. It will be understood that, while a particular anatomical site is shown by way of illustration, other locations may be used for vascular access. When not in use for dialysis, device 300 and cap 390 may be protected against day to day accidental impacts and the like by protective cover 420. Cover 420 includes tab or strap portions 420a, 420b (not shown to scale) which may be used to secure the cover 440 in place on the arm 270 over the cap 390. In one embodiment, the tab or strap portions 420a, 420b form an armband. Alternatively, a separate armband (not shown) may be provided for securing the protective cover 420 in place.

For clarity, in Figure 7, cap 390 and protective cover 420 are shown "exploded" away from the device 300 but it will be understood that while in use they are maintained in contiguity with the device 300 and patient.

It is advantageous to locate an inductive antenna 440 and, optionally, other elements of the overall signal processing generally indicated as 520 in Figure 8, within the cover 420. With cover 420 in use, antenna 440 is located over the proximal end of the device 300 and in communication with antennas 380 and/or 400 or 410 for reception of data from the sensors and interface electronics of cartridge 330. Such an inductive antenna 440 may also include a ferrite core and/or be tuned as described above. Connection to data processor 450 may be provided as shown by cable 455 or the connection may be wireless using any suitable wireless communications protocol. In the latter case, cover 420 will also be provided with an easily replaceable or rechargeable battery.

Optionally, power transmission from the armband or cover 420 may also be provided via the inductive antenna coupling for recharging battery 365 within cartridge 330.

Upon insertion of dialysis cannula 280 through septum 340, it is preferable that external transmission of signals from the sensors cease, averting potential incorrect readings or interference with the dialysis apparatus. Insertion of the cannula 280 into the cavity 335 of the cartridge 330 can be detected by the inclusion of a magnetic component (not shown) in the cannula assembly that will locate in close proximity to inductive antenna coil 380 altering its electrical impedance, the magnetic component and its interaction with the inductive antenna coil 380 effectively disabling the transmission of data from the cartridge 330.

Processor 450 may be worn by the patient with a wired connection 455 to module 520 as shown in Figure 7, or alternatively, placed at a location remote from the patient with a wireless connection. This capability is advantageous for 24/7 monitoring allowing placement of the processor at the patient's bedside, for example.

The processor 450 may further communicate wirelessly using, for example, a Bluetooth communications link (Bluetooth is a trademark of the Bluetooth Special Interest Group.) as indicated by transmit and receive arrows 465a, 465b, with a reading device 460 which transmits data to a central remote location (not shown). In this case, the reading device 460 may be a "smart" phone or other similar device which can both receive data from the remote processor 450 and transmit data to the central remote location, for example, a doctor's surgery, clinic, hospital or other monitoring station.

It will readily be understood that the processor 450 and reading device 460 may be integrated into a single device (not shown). In this case, the single device may be a "smart" phone running an appropriate application. If this is the case, the connection between the antenna 440 and electronics 540 in the cover 420 and the single device may be wireless.

Referring now to Figure 8, a block diagram of the operational electronic components of a blood monitoring system 500 using the device 300 of the present invention is shown. As before, components that have been previously been described with reference to Figures 2 to 7 bear the same reference numerals. However, since the operating function remains the same in each embodiment, for simplicity coil 380 and any one or several in combination of the coils and antennae 400 and 410 and the cores 385 and 405 as shown in Figure 3 are collectively referenced as antenna assembly 610 in Figure 8.

In outline, blood monitoring system 500 comprises, in the exemplary glucose monitoring case, sensor set 375, as previously described, located on the distal end 340a of the septum 340 portion of cartridge 330; integrated circuit 370; battery 365; antenna assembly 610, all forming parts of the port and components; external antenna 440; external receiver/transmit module 520; an interface assembly 450; and a reading device 460, for example, a "smart" phone. The blood monitoring system 500 also includes within cartridge 330 the flexible circuit 360 (not shown in Figure 8) which provides interconnections between components of the system as will be described in more detail below.

In use, due to their positioning, the electrodes 375a, 375b, 375c of the sensor set 375 are in contact with the blood flow 205 within the AVG 200 (Figure 2). An electrical potential is maintained on the reference electrode 375c and the counter electrode 375b by a regulating circuit 550. Circuit element 560 senses the current flow through sensing electrode 375a, the electrical current being representative of the blood glucose concentration. As shown, interconnections 510a, 510b, 510c, connect the electrodes 375a, 375b, 375c respectively to the sensor circuit element 560 and regulating circuit 550.

For sensor and circuit shielding, common connections of regulating circuit 550 and circuit element 560 are electrically connected via septum surface electrode 375d and shoulder 325 to port body 310 (Figure 3 and Figure 5).

Circuit 560 connected to sensing electrode 375a provides an output analog signal 565 to an analog-to-digital converter (ADC) 570. The ADC 570 converts the output analog signal from the sensor circuit element 560 into a digital signal 575 which is encoded with time interval data or other identifying information in encoder 580. The encoded digital signal is stored in a non-volatile memory (NVM) 590.

In some sensor arrangements, the ADC 570 may compensate for non-linearities or signal offsets in sensor operation. For example, a look-up table may be provided at manufacture and may be modified during operation by signals from the transmit/receive module 520. Such modification may comprise recalibration of the sensor circuit element 560 using independent blood glucose measurements taken by the patient or at a clinic or other monitoring facility.

A data transmission circuit 600 is connected to process the encoded digital signal from the NVM 590 for transmission to the external antenna 440 and module 520 as indicated by signal 615a. The data transmission circuit 600 is connected to antenna assembly 610 (comprising one of more of the components listed above) via a transmit/receive switch 620, connection 625 between the transmit/receive switch 620 and the antenna 610 (collectively 380 and other components as above) being provided by the flexible circuit 360 (Figures 3 and 4).

Also connected to the transmit/receive switch 620 is a data receive circuit 630 for receiving data transmitted to the antenna 610. Transmit/receive switch 620 provides the additional function of protecting the data receive circuit 630 from overload by the data transmission circuit 600. Further protection is provided by 620 limiting energy transferred to other components within cartridge 330 from unwanted external signals such as those from a cellular phone or a magnetic resonance imaging (MRI) scanner. Additional selectivity against such signals may be realized by inductive antenna tuning as described above.

In one embodiment of the blood glucose measurement system 500 in accordance with the present invention, the integrated circuit 370 is paired or matched with the transmit/receive module 520. This may be implemented by encoding the transmitted signals 615a to the transmit/receive module 520 by the antenna 610 and the transmit/receive module 520 being programmed to receive only signals that have the appropriate encoding. This has the advantage that patient data integrity is maintained and communication interference with other patients also fitted with vascular port access devices 300 (Figures 2 to 5) is prevented.

Data transmission circuit 600 is controlled by a timing circuit 640 which is programmed to provide sensor activation, data storage and data transmission as predetermined intervals chosen to sample sufficiently the expected temporal variations in blood conditions.

The data receive circuit 630 allows for the reception of set up instructions via signal 615b from the transmit/receive module 520 to control sample timing intervals or to initiate a readout of stored data from NVM 590. This allows, for example, access to data obtained during periods when the transmit/receive module 520 is non-operational and for repeat transmission requests in the case of incomplete data transfer from the cartridge 330 within the vascular access port device 300 (Figure 3) to the transmit/receive module 520. Such data may include indications of the status of components within the cartridge 330, for example, the charge level of the battery 365. Received signal 615b is preferably also encoded and a recognition template of the encoding information is stored in the NVM 590. The recognition template may be preset during manufacture of the cartridge 330, or may be set when the cartridge 330 is replaced as will be described below in more detail.

The battery 365 may operate to maintain a minimum "keep alive" power only, and, preferably, it is connected to timing circuit 640 and to data receive circuit 630. Power use during events initiated by timing circuit 640 or on reception of a received signal 615b from the transmit/receive module 520 is regulated by power control circuit 650. Power control circuit 650 controls and activates the encoding circuit 580, the data transmission circuit 600, the sensor circuit 560, the regulating circuit 550, and the ADC 570 respectively, via interconnections 660a, 660b, 660c, 660d as shown, in accordance with each sampling or readout event.

In order to minimize the size of the interconnections within the cartridge 330, most or all the circuitry required for the functional components of the cartridge 330 are provided by the integrated circuit 370. Although the cartridge 330 is described as being used for blood glucose measurements, it will readily be appreciated that the electrode set 375, sensor circuit 560 and regulating circuit 550 can be configured for a wide variety of conditions within the blood measurement or monitoring system 500 in accordance with the present invention.

Turning now to the transmit/receive module 520, this comprises processing elements coupled to the antenna 440 in the cover 420, which, in turn, are in communication with the interface assembly 450. In a typical arrangement, the transmit/receive module 520 is worn by the patient or user of the blood monitoring system 500.

A receiver module 675 is adapted to detect and filter signals 615a transmitted from the implanted sensor by the antenna 610 as described above, a buffer 670 connected to receiver module 675, and a transmitter module 680 adapted to provide signal 615b as needed.

Buffer 670 provides temporary storage for received signals from the implanted sensor. This allows reconstruction of incomplete transmissions and holding of data bursts from the readout of data stored in NVM 590 of the integrated circuit 370.

The transmit/receive module 520 is connected to interface assembly 450 by wired connection 455 (or, as described above, by means of a wireless connection). The interface assembly 450 includes a microprocessor 690 which controls the operation of the transmitter module 680 to excite antenna 440 with the transmit signal 615b for set up and interrogation of the integrated circuit 370 implanted in the vascular access port device 300 as described above.

Interface assembly 450 may optionally include an interface 700 to receive signals from a physiological monitor 710 which provides signals indicative of pulse rate and respiration, for example, of a patient using the blood measurement system 500. It will be appreciated other physiological signals can also be monitored by the monitor 710 and it is not limited to pulse rate and respiration.

As shown receiver 675, transmitter 680, buffer 670 and interface 700 are functionally connected to microprocessor 690. The microprocessor 690 operates to provide: sequencing and control for transmit/receive module 520; interface and receipt of glucose levels or other data via buffer 670; receipt of other physiological conditions from interface 700; programmed calculation of a medical record from the measured sensor data; an external interface means 720, such as, provided, for example, by a universal serial bus (USB) for setting operating conditions of the overall system by a physician or other medically trained person; input of calibration data obtained independently, for example, by the patient or clinic using a separate glucose meter (such calibration data may be transmitted to the implanted memory of the integrated circuit 370 and applied as described above); and management of power consumption of battery 730 in the interface assembly 450.

During calculation of the medical record from the glucose or other readings from the patient, the microprocessor 690: detects and rejects corrupted data; provides data averaging and other filtering means to reduce transmission noise; calculates and maintains sensed glucose level data over an extended time; extrapolates shorter term analyte level trends and provides a patient audible or other alarm for incipient serious conditions such as hypoglycemia; and may maintain a local indication of sensed conditions via a display 740 within the interface assembly 450.

Assembly 450 may also include a charger (not shown) for battery 730. In many cases, it will be convenient to use USB interface 720 for charging power.

For extended use periods of cartridge 330, a modified configuration may comprise a rechargeable battery 365. As required, transmit/receive module 520 generates a second signal (not shown) at a higher power level than needed for communication only, and well known rectifying and charging control means may be added to the integrated circuit 370.

For everyday patient use, antenna 440 and those functions contained within transmit/receive module 520 may advantageously be packaged within the cover 420 as described above also providing mechanical protection to the access port site. Processor 690 and the other components contained separately in interface assembly 450 then provide power and communication via connection 460. Most often, it will be preferable that the connection be provided wirelessly and that transmit/receive module 520 further comprises a battery (not shown). By these means, location of the microprocessor 690 and associated functions within assembly 450, including display and alarms, is flexible, for example, worn on the patient clothing or placed on a bedside table for overnight monitoring.

It will be further preferred that the cover 420 can be readily laundered without damage to the antenna 440 or transmit/receive module 520. Multiple interchangeable covers may be provided without confusion since transmit/receive module 520 does not itself create or sense signal coding but simply transfers signals without reference to the specific data coding provided by microprocessor 690.

It will readily be understood that, while this description makes particular reference to blood glucose monitoring, additional sensors may further be provided on and/or within cartridge, either individually or severally, for other blood analytes, including without limitation: urea, creatinine, potassium, sodium, hemocrit and pH. As a particular example, monitoring potassium and creatinine jointly and transmission of these data to the patient's physician would provide an indication of patient condition between dialysis sessions and a prompt for patients using home hemodialysis. Physical measurements may also be made, including blood pressure or temperature, within the graft or fistula as an indication of flow patency.

Various modifications to the embodiments described above may occur to those skilled in the art without departing from the intent and scope of the invention. The various embodiments described above can be combined to provide further implementations of the blood measurement/monitoring system in accordance with the present invention.

These and other changes can be made to the embodiments in light of the above description. In general, the terms used in the claims should not be construed to be limited to the specific embodiments disclosed in the description and defined by the claims, but should be construed to include all possible embodiments along with the full scope of equivalents to which such claims are entitled.

## Claims

1. A cartridge (330) for use in a vascular access port device (300) operatively connected to a vascular component (200) of a patient, the cartridge having a distal end (330a) and a proximal end (330e), the cartridge comprising:-
an internal cavity (335, 335a, 335b) adapted to receive a cannula for connection to an external dialysis machine; and
a re-sealable element (340) located at the distal end (330a) of the cartridge (330) operable for providing cannula access to the vascular component when located in the vascular access port device, the re-sealable element having an external surface in contact with blood in the vascular component (205);
**characterized in that** the cartridge further comprises a sensor arrangement (375) located on the external surface of the re-sealable element (340), the sensor arrangement (375) being adapted for generating electrical signals indicative of measurements taken thereby; an integrated circuit (370) for processing the generated electrical signals; and an inductive coil element (380) for transmitting the processed electrical signals to an external location.

2. A cartridge according to claim 1, further comprising a flexible circuit (360) for providing connections between the electrode set (375), the integrated circuit (370), and the inductive coil element (380).

3. A cartridge according to claim 1 or 2, further comprising a power supply (365) connected to the integrated circuit (370).

4. A cartridge according to any one of the preceding claims, wherein the inductive coil element (380) is located in an upper portion (335b) of the cavity (335) around an inner wall (335c) thereof.

5. A cartridge according to any one of the preceding claims, further comprising a substantially rigid insert (350) located within a lower portion (335a) of the cavity (335) on the inner wall (335c) thereof.

6. A cartridge according to any one of the preceding claims, wherein the sensor arrangement (375) comprises an electrode set with a sensing electrode (375a), a counter electrode (375b) and a reference electrode (375c).

7. A cartridge according to any one of the preceding claims, further comprising an electrical contact for forming a common connection with the vascular access port device and for shielding the sensor arrangement (375) against interfering signals.

8. A vascular access port system (300) comprising:-
an implantable body portion (310) having a distal end (310a) and a proximal end (310d) and being operable for being percutaneous implantation with the distal end defining an opening into a vascular component (200);
a retention portion (320a, 320b) formed on an outer wall (310a) of the implantable body (310) which is adapted for anchoring the implantable body in place relative to the vascular component (200); and
a cartridge (330) according to any one of the preceding claims located within the implantable body portion (310), the sensor arrangement (375) being located on an external surface of the re-sealable element (340) and positioned at the distal end (310a) of the implantable body portion (310) for contact with blood flow (205) through the vascular component (200).

9. A vascular port access system according to claim 8, further comprising a mechanically compliant layer (355) on outer wall (310c) of the implantable body (310).

10. A vascular port access system according to claim 9, further comprising an infection resistant tissue interface layer (356) formed over the mechanically compliant layer (355) on the outer wall (310c) of the implantable body (310).

11. A vascular port access system according to any one of claims 8 to 10, further comprising a cap (390) locatable on the implantable body portion (310), the cap (390) having a rim portion (390a) operable for engaging with a groove portion (310h) formed at the proximal end (310d) of the implantable body (310).

12. A vascular port access system according to claim 11, wherein the cap (390) includes an inductive antenna coil (400), the inductive antenna coil (400) being alignable with the inductive antenna coil (380) in the cartridge (300) when the cap (390) is attached to the implantable body portion (310).

13. A vascular port access system according to claim 12, wherein the cap (390) comprises a high magnetic permeability liner (405) associated with the inductive antenna coil (400).

14. A vascular port access system according to any one of claims 8 to 13, wherein the cartridge (330) is replaceable.

15. A vascular port access system according to claim 14, wherein a passive cartridge is used when sensing of blood conditions is not required.

16. A vascular port access system according to claims 14 or 15, further comprising a balloon catheter for sealing port (310b) during replacement of the cartridge, the balloon catheter penetrating the septum (340) and inflating beyond the distal end (340a) thereof.

17. A blood monitoring system (500) for monitoring blood parameters of a hemodialysis patient, the system comprising:-
a vascular port access system (300) according to any one of claims 8 to 16 implanted in the hemodialysis patient and operable for providing signals indicative of blood parameters of the patient; and
a processor (450, 460; 520) operable for receiving the signals from the vascular port access system (300), for processing the received signals to provide data relating to the blood parameters, and for transmitting the data to a remote monitoring station.

18. A blood monitoring system according to claim 17, wherein the processor (450, 460; 520) comprises an interface assembly (450).

19. A blood monitoring system according to claim 18, wherein the interface assembly (450) is wirelessly connected to the vascular access port system (300).

20. A blood monitoring system according to any one of claims 17 to 19, wherein the processor (450, 460; 520,) comprises a transceiver device (460) connected to the interface assembly (450 by a communications link (465a, 465b).

21. A blood monitoring system according to claim 20, wherein the transceiver device (460) comprises a smart phone.
